# EUROPÄISCHE PATENTANMELDUNG

(11) **EP 1 273 567 A2**
(43) Veröffentlichungstag der Anmeldung: **08.01.2003**
(21) Anmeldenummer: 02013649.5
(22) Anmeldetag: 20.06.2002
(51) Int. Cl.: C07C 233/38, A61K 7/075, C08F 20/60

(54) **Betainester**

(30) Priorität: 03.07.2001 DE 10132173
(71) Anmelder: Goldschmidt AG, 45127 Essen (DE)
(72) Erfinder: Hell, Kerstin, 45326 Essen (DE); Leidreiter, Holger, Dr., 45529 Hattingen (DE); Tomuschat, Philipp, Dr., 45128 Essen (DE); Weyershausen, Bernd, Dr., 45127 Essen (DE)

(57) **Zusammenfassung**

Gegenstand der Erfindung sind Verbindungen der allgemeinen Formel (I) 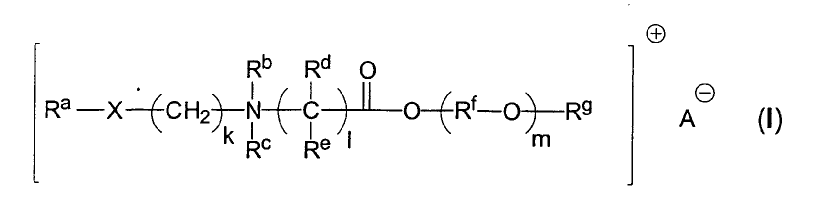 in denen
R^{a} ein ethylenisch ungesättigter, wenigstens eine Carbonylfunktion enthaltender Rest mit 1 bis 5 C-Atomen ist sowie deren Homopolymeren und Copolymeren mit Verbindungen der allgemeinen Formel (II) 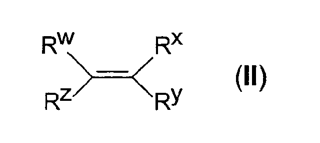 und deren Verwendung in Haarkonditioniermitteln.

## Beschreibung

Die Erfindung betrifft neue polymerisierbare Betainester sowie aus diesen herstellbare Polymere.

Durch regelmäßiges Waschen von Haaren mit entfettenden Tensiden sowie häufiges Bleichen, Dauerwellen und Färben kommt es zu einer Schädigung der Haarstruktur. Vor diesem Hintergrund haben Haarbehandlungsmittel bzw. Konditioniermittel mit kämmbarkeitsverbessernden und pflegenden Eigenschaften eine erhebliche Bedeutung erlangt. Gleichzeitig spielen ästhetische Faktoren wie z. B. Geruch eine immer wichtigere Rolle. Aus diesem Grund wird in vielen Haarpflegeformulierungen zusätzlich zu den konditionierenden Komponenten ein Parfümöl verwendet.

Konditionierende Formulierungen dienen dem Schutz vor Schädigung, der symptomatischen Reparatur von Schädigungen und der Überdeckung von Schädigungen. Als Konditioniermittel in solchen Formulierungen werden allgemein kationische oder aminofunktionelle Stoffe eingesetzt. Diese Stoffe können mit anionischen Zentren auf der Haaroberfläche wechselwirken und substantiv auf diese Oberfläche aufziehen.

Gebräuchlich als Konditioniermittel sind beispielsweise Cetyltrimethylammoniumsalze, Distearyldimethylammoniumsalze, Fettsäureester von Triethanolmethylammoniumsalzen, methylierte Imidazoliniumsalze und andere Produkte. Auch Betainester werden für die Verwendung in Haar- und Körperpflege- sowie Reinigungsprodukten vorgeschlagen. Sie sollen unter anderem für die Herstellung von besonders milden Haar- und Hautkonditioniermitteln sowie Shampoos verwendet werden, wie beispielsweise beschrieben in der DE-A-35 27 974, DE-A-43 09 567, EP-A-0 367 939 oder EP-A-0 507 003. Die Betainester werden jedoch in Bezug auf ihre konditionierenden Eigenschaften im Vergleich zu den o. g. gebräuchliche Konditioniermitteln als wenig effektiv angesehen.

Es ist bekannt, dass kationische oder aminofunktionelle Konditioniermittel durch die Verwendung von Parfümölen in eine kosmetisch/geruchlich akzeptable Formulierung gebracht werden müssen. Insbesondere längere Zeit nach Anwendung der konditionierenden Formulierung lässt die Wirkung des Parfümöls jedoch nach und der fettartige und oder aminische Geruch des Konditioniermittels wirkt sich negativ aus.

Aufgabe der vorliegenden Erfindung war es, neue Betainester bzw. Betainesterpolymere zur Verfügung zu stellen, die bei gutem Aufzugsverhalten auf das Haar diesem einen lang anhaltenden angenehmen Duft verleihen. Überraschenderweise weisen die erfindungsgemäßen Verbindungen zusätzlich sehr gute konditionierende Eigenschaften auf. Auch die nicht duftalkoholfunktionalisierten polymeren Betainester besitzen konditionierende Eigenschaften.

Gegenstand der Erfindung sind daher polymerisierbare Betainester der allgemeinen Formel (I) 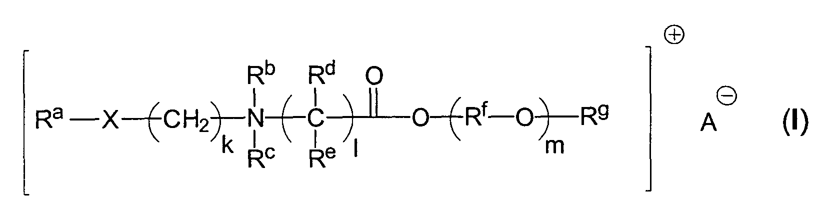 in denen
- R^{a}: ein ethylenisch ungesättigter, wenigstens eine Carbonylfunktion enthaltender Rest wie zum Beispiel Acryloyl, Methacryloyl, Maleinoyl oder Itaconoyl ist;
- X: ein Sauerstoffatom, -N(CH₃)- oder -NH- ist;
- R^{b}, R^{c}: unabhängig voneinander gegebenenfalls verzweigte Alkylreste mit 1 bis 4 C-Atomen sind, wobei diese Heteroatomsubstituenten, insbesondere O, S, N, P, enthalten können;
die Reste
- R^{d}, R^{e}: unabhängig voneinander ausgewählt werden aus Wasserstoff (H), gegebenenfalls verzweigten Alkylresten mit 1 bis 4 C-Atomen, gegebenenfalls substituierten Aryl- oder Benzylresten sowie -CH₂COOH, -CH₂COOR, -CH₂CH₂COOH, -CH₂CH₂COOR;
- R^{f}: ein gegebenenfalls Mehrfachbindungen enthaltender verzweigter und/oder substituierter und/oder zyklischer Kohlenwasserstoffrest mit 1 bis 10, vorzugsweise 2 oder 3 Kohlenstoffatomen ist, oder ein Styrolrest ist oder ausschließlich aus Ethylen- oder Propylen- oder Butylen- oder Styrolresten aufgebaut ist, oder ein die genannten Reste enthaltendes Block-Copolymer oder statistisch aufgebautes Copolymer ist;
- R^{g}: ein gegebenenfalls verzweigter, gegebenenfalls Doppelbindungen enthaltener, gegebenenfalls zyklischer Kohlenwasserstoffrest mit 1 bis 22 C-Atomen ist, wobei R^{g} ein gegebenenfalls verzweigter, gegebenenfalls Doppelbindungen enthaltener Kohlenwasserstoffrest ist, wenn m = 0 ist und R^{g} H sein kann, wenn m > 0 ist;
- k, l: unabhängig voneinander 1 bis 4 sind, wobei k vorzugsweise 2 oder 3 und l vorzugsweise 1 ist und
- m: einen Wert zwischen 0 bis 100, vorzugsweise 0 bis 40 hat;
- A⁻: ein Anion ist.

Weitere Gegenstände der Erfindung sind Homopolymerisate, hergestellt aus polymerisierbaren Betainestern der allgemeinen Formel (I) sowie Copolymerisate hergestellt aus polymerisierbaren Betainestern der allgemeinen Formel (I) und geeigneten Comonomeren der allgemeinen Formel (II). 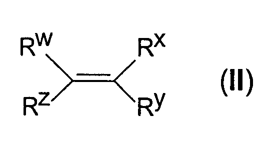 in denen
- R^{x} und R^{y}: H sind,
- R^{w}: H oder CH₃ ist und
- R^{z}: ein wenigstens eine Carbonylgruppe enthaltender Rest, wie zum Beispiel -C(O)OR, -C(O)NR'R'' ist, wobei R, R' und R" H oder gegebenenfalls Mehrfachbindungen enthaltende, lineare oder verzweigte und/oder zyklische und/oder substituierte und/oder Halogenatome enthaltende und/oder Heteroatome enthaltende und/oder Carbonylgruppen enthaltende Kohlenwasserstoffreste mit 1 bis 18 C-Atomen sind;
oder in denen
- R^{w} und R^{x}: H sind,
- R^{y} und R^{z -}: eine Carbonylgruppe enthaltende Reste, wie zum Beispiel -C(O)OR, -C(O)NR'R" sind, wobei R, R' und R" H oder gegebenenfalls Mehrfachbindungen enthaltende, lineare oder verzweigte und/oder zyklische aliphatische oder aromatische und/oder substituierte und/oder Halogenatome und/oder Heteroatome enthaltende Kohlenwasserstoffreste mit 1 bis 18 C-Atomen sind;
oder in denen
- R^{w}, R^{x} und R^{y}: H sind und
- R^{z}: ein gegebenenfalls halogenatom- und/oder heteroatomsubstituierter, lineare und/oder verzweigte Alkylsubstituenten enthaltender Aromat oder Heteroaromat ist;
oder in denen
- R^{w}, R^{x} und R^{y}: H sind und
- R^{z}: -(CH₂)ₐ-OR''' ist, wobei R''' H oder ein gegebenenfalls Carbonylgruppen enthaltender Alkylrest mit 1 bis 22 C-Atomen oder ein ausschließlich aus Ethylen- oder Propylen- oder Butylen- oder Styroloxid aufgebauter Polyether ist, der ein die genannten Reste enthaltendes, blockweise oder statistisch aufgebautes Copolymer darstellt und a 0 oder 1 ist.

Geeignete Comonomere werden beispielsweise ausgewählt aus folgenden repräsentativen Gruppen:
Ethylenisch ungesättigte, Carboxylgruppen enthaltende Verbindungen wie zum Beispiel Acrylsäure, Methacrylsäure, Maleinsäure, Maleinsäureanhydrid, Maleinsäurehalb- und -diester der Itaconsäure;
C₁-C₁₈-Acrylsäure-, Methacrylsäureester bzw. Maleinsäurehalb- oder -diester wie zum Beispiel Methylacrylat, Ethylacrylat, Propylacrylat, n-Butylacrylat, i-Propylacrylat, t-Butylacrylat, n-Hexylacrylat, Methylmethacrylat, Ethylmethacrylat, Propylmethacrylat, i-Propylmethacrylat, n-Butylmethacrylat, n-Hexylmethacrylat, n-Dodecylmethacrylat;
Polyethylenglykol- und Polypropylenglycolacrylate und -methacrylate;
C₂-C₁₈-Alkoxyester der Acryl- sowie Methacrylsäure wie zum Beispiel Methoxyethylacrylat, Ethoxyethylacrylat, Methoxyethylmethacrylat, Ethoxyethylmethacrylat, Methoxybutylacrylat, Methoxybutylmethacrylat, Ethoxybutylacrylat, Ethoxybutylmethacrylat;
C₂-C₈-Hydroxyalkylacrylate und -methacrylate wie zum Beispiel Hydroxyethylacrylat, Hydroxyethylmethacrylat, Hydroxyproylacrylat, Hydroxyproylmethacrylat, Hydroxybutylacrylat, Hydroxybutylmethacrylat;
Vinylverbindungen wie zum Beispiel Styren, α-Methylstyren, Vinyltoluen, p-Chlorstyren, Vinylacetat und andere Vinylester und auch Vinylether, Vinylpolyether, Vinylalkohol, halogenierte Vinylverbindungen, Allylvinylketon, 4-Vinylpyridin, N-Vinylpyrrolidon, Vinylimidazol;
Amide der Acryl- und Methacrylsäure wie zum Beispiel Methacrylamid, N,N-Dimethylacrylamid, N,N-Dimethylmethacrylamid, N,N-Dimethylaminopropylacrylamid, N,N-Dimethylaminopropylmethacrylamid;
Diallyldimethylammoniumchlorid (DADMAC) ;
Allylpolyether.

Diese Auswahl stellt jedoch keine Einschränkung bezüglich der in Frage kommenden Comonomeren dar.

Weitere Gegenstände der Erfindung sind gekennzeichnet durch die Ansprüche.

Überraschenderweise wurde nun gefunden, dass die neuen erfindungsgemäßen polymerisierbaren Betainester und polymeren Betainester, die im Falle der Homopolymerisate aus den monomeren polymerisierbaren Betainestern der allgemeinen Formel (I) hergestellt sind und im Falle der Copolymerisate aus polymerisierbaren Betainestern der allgemeinen Formel (I) und geeigneten Comonomeren der allgemeinen Formel (II) hergestellt sind, konditionierende Eigenschaften besitzen, wobei insbesondere im Falle der duftalkoholfunktionalisierten Betainesterpolymeren der angenehme Geruch als besonders positive Eigenschaft herauszustellen ist.

### Verbindungen der allgemeinen Formel (I)

Die erfindungsgemäßen monomeren, polymerisierbaren Betainester der allgemeinen Formel (I) stellen neue Stoffe dar, welche nach den bekannten Methoden des Standes der Technik zur Herstellung bekannter Betainester hergestellt werden können. Monomere Betainester der allgemeinen Formel (I) können dann nach den bekannten Methoden des Standes der Technik unter Verwendung der üblichen Initiatoren wie Kaliumperoxodisulfat oder Diazoverbindungen radikalisch homo- oder copolymerisiert werden (siehe auch D. Braun, H. Cherdron, H. Ritter, Praktikum der Makromolekularen Stoffe, Wiley-VCH, Weinheim, 1999).

Im Allgemeinen wird so verfahren, dass in einem ersten Schritt zunächst die Halogencarbonsäure, vorzugsweise Monochloressigsäure, (10-100 % Überschuss, vorzugsweise 10 %) mit der entsprechenden Alkoholkomponente HO-(R^{f}-O)ₘ-R^{g} gegebenenfalls unter Mitverwendung von Katalysatoren verestert wird, indem die beiden Reaktionspartner unter Stickstoffatmosphäre auf 80 bis 200 °C, vorzugsweise 100 bis 140 °C, besonders bevorzugt ca. 120 °C, erhitzt werden. Das entstehende Reaktionswasser wird hierbei kontinuierlich über eine Destillationsbrücke abdestilliert. Die Veresterung dauert im Allgemeinen etwa drei Stunden und wird abgebrochen, wenn kein Reaktionswasser mehr abdestilliert. Anschließend wird die überschüssige Monochloressigsäure im Vakuum bei Drücken von vorzugsweise kleiner 1 mbar destillativ entfernt. Die Reinheit der Produkte wird unter anderem über den Gehalt an Chlor und NMR-Spektroskopie überprüft. Im zweiten Schritt erfolgt die Quaternierung von ethylenisch ungesättigten Carbonsäureamidaminen mit dem Monochloressigsäureester. Hierzu wird der Monochloressigsäureester zusammen vorzugsweise mit einem Amidamin der Formel R^{a}-NH-(CH₂)ₖ-NR^{b}R^{c} in Gegenwart von 0,01 Gew.-% bis 0,5 Gew.-%, vorzugsweise 0,1 Gew.-%, eines Radikalfängers (Inhibitor) unter Stickstoffatmosphäre für etwa drei bis sechs, vorzugsweise vier, Stunden auf 40 bis 80 °C, vorzugsweise 60 °C, erhitzt.

Die Homopolymerisation von Betainestern wird im Allgemeinen folgendermaßen durchgeführt: zunächst wird der Betainester unter Stickstoffatmosphäre vorgelegt und ein Lösungsmittel, vorzugsweise Wasser oder Isopropanol, zugegeben. Anschließend wird auf 60 bis 120 °C, vorzugsweise 80 bis 100 °C, besonders bevorzugt 90 °C im Falle von Wasser als Lösungsmittel und 80 °C im Falle von Isopropanol als Lösungsmittel erhitzt, wobei der Betainester sich langsam vollständig löst.

Bei der Verwendung von Wasser als Lösungsmittel werden nach Erreichen der Reaktionstemperatur 1 bis 5 Mol-%, vorzugsweise 2 Mol-% eines in Wasser löslichen Radikalstarters, vorzugsweise Kaliumperoxodisulfat, gelöst in Wasser, unter Rühren über einen Zeitraum von 0,5 bis 2 Stunden, vorzugsweise 1 Stunde zugetropft. Anschließend wird für weitere 1 bis 4 Stunden, vorzugsweise 2 Stunden bei der jeweiligen Temperatur gerührt, und das Wasser anschließend im Vakuum abdestilliert.

Bei der Verwendung von Isopropanol als Lösungsmittel werden nach Erreichen der Reaktionstemperatur 0,05 bis 0,5 Mol-%, vorzugsweise 0,1 bis 0,2 Mol-%, eines Diazo-Radikalstarters, gelöst in Isopropanol, unter Rühren über einen Zeitraum von 1 bis 5 Stunden, vorzugsweise 2 Stunden zugetropft. Anschließend wird für weitere 1 bis 3 Stunden bei Reaktionstemperatur gerührt. Anschließend wird zur Vervollständigung der Polymerisation die ein- bis dreifache, vorzugsweise die eineinhalbfache Menge Diazo-Radikalstarter bezogen auf die erste Zugabe, gelöst in Isopropanol, über einen Zeitraum von 0,5 bis 1,5 Stunden, vorzugsweise 30 bis 45 Minuten zugetropft und weitere 1 bis 3 Stunden, vorzugsweise 2 Stunden, bei Reaktionstemperatur gerührt. Unabhängig vom verwendeten Lösungsmittel wird dieses nach vollendeter Reaktion im Vakuum abdestilliert.

Copolymerisationen werden in einem Lösungsmittel, vorzugsweise in Isopropanol, unter Schutzgasatmosphäre durchgeführt, wobei das Lösungsmittel vorgelegt wird und Lösungen der Comonomeren gleichzeitig in das Reaktionsgefäß zugetropft werden. Hierbei ist der Radikalstarter, vorzugsweise eine Diazoverbindung, in einer der Lösungen, die eines der Comonomeren enthält, gelöst. Ansonsten sind die Reaktionsbedingungen denen der Homopolymerisation mit Diazoverbindungen analog.

Erfindungsgemäß bevorzugt werden Verbindungen, in welchen der Rest R^{a} -C(O)-CH=CH₂ ist und insbesondere die Reste R^{b} und R^{c} gleichzeitig -CH₃ sind und die Reste R^{d} und R^{e} gleichzeitig H sind
und/oder Verbindungen, in denen k = 2 oder 3 ist und l = 1 ist
und/oder Verbindungen, in denen m = 0 ist, vorzugsweise Verbindungen, in denen R^{a} -C(O)-CH=CH₂ ist, R^{b} und R^{c} -CH₃ sind, R^{d} und R^{e} gleichzeitig H sind, R^{f} ein Ethylenrest oder Propylenrest und k = 3, l = 1, m = 0 bis 40 und R^{g} ein Alkylrest mit 1 bis 22 C-Atomen oder Wasserstoff ist.

Weiterhin sind Verbindungen der allgemeinen Formel (I) bevorzugt, in denen R^{f} ein Styrol- oder Butylenrest sein kann oder vorzugsweise ein Propylenrest, besonders bevorzugt sind Verbindungen der allgemeinen Formel (I), in denen R^{f} ein Ethylenrest ist, wobei R^{f} ausschließlich aus Ethylen- oder Propylen- oder Butylen- oder Styrolresten aufgebaut sein kann, aber auch ein, die genannten Reste enthaltendes Block-Copolymer oder statistisch aufgebautes Copolymer,
und/oder Verbindungen, in denen R^{g} ein Alkylrest oder Alkenylrest mit 8 bis 18 C-Atomen ist wie vorzugsweise der Rest eines nach bekannten Verfahren herstellbaren Fettalkohols auf Basis natürlicher Fettsäuren,
und/oder Verbindungen, in denen m = 0 und R^{g} ein Duftalkohol ist.

Als Duftalkohole im Sinne der vorliegenden Erfindung können alle "aktiven Alkohole" verwendet werden, welche eingearbeitet in Formulierungen in Konzentrationen von ca. 0,01 bis ca. 10 Gew.-% diesen einen angenehmen Duft verleihen. Verbindungen dieser Art werden beispielsweise beschrieben in der EP-A 0 799 885, EP-A 0 771 785, WO 96/38528 (PCT/US96/06758), US-A-5 958 870. Aus dieser Klasse werden erfindungsgemäß bevorzugt mitverwendet: 2-Phenoxyethanol, Phenylethylalkohol, Geraniol, Citronellol, Hydroxycitronellal, Farnesol, Menthol, Eugenol, Vanilin, Ethylvanilin, cis-3-Hexenol, Nerol, α-Terpineol, Linalool, Tetrahydrolinalool, 1,2-Dihydromyrcenol, 3-Methyl-5-phenyl-1-pentanol, 2,4-Dimethyl-3-cyclohexen-1-methanol. Diese Auswahl stellt jedoch keine Einschränkung bezüglich der in Frage kommenden Duftalkohole dar.

Als Fettsäuren für die Herstellung von Fettalkoholen werden - einzeln oder in Mischungen - Fettsäuren wie Capronsäure, Caprylsäure, Caprinsäure, 2-Ethylhexansäure, Laurinsäure, Myristinsäure, Palmitinsäure, Palmitoleinsäure, Isostearinsäure, Stearinsäure, Hydroxystearinsäure (Ricinolsäure), Dihydroxystearinsäure, Ölsäure, Linolsäure, Petroselinsäure, Elaidinsäure, Arachinsäure, Behensäure und Erucasäure, Gadoleinsäure sowie die bei der Druckspaltung natürlicher Fette und Öle anfallenden technischen Mischungen wie Ölsäure, Linolsäure, Linolensäure, und insbesondere Rapsölfettsäure, Sojaölfettsäure, Sonnenblumenölfettsäure, Tallölfettsäure mitverwendet. Geeignet sind prinzipiell alle Fettsäuren mit ähnlicher Kettenverteilung.

Der Gehalt dieser Fettsäuren bzw. Fettsäureester an ungesättigten Anteilen, wird -soweit dies erforderlich istdurch die bekannten katalytischen Hydrierverfahren auf eine gewünschte Jodzahl eingestellt oder durch Abmischung von vollhydrierten mit nichthydrierten Fettkomponenten erzielt.

Die Jodzahl, als Maßzahl für den durchschnittlichen Sättigungsgrad einer Fettsäure, ist die Jodmenge, welche von 100 g der Verbindung zur Absättigung der Doppelbindungen aufgenommen wird.

Vorzugsweise werden teilgehärtete C_{8/18}-Kokos- bzw. Palmfettsäuren, Rapsölfettsäuren, Sonnenblumenölfettsäuren Sojaölfettsäuren und Tallölfett-säuren, mit Jodzahlen im Bereich von ca. 80 bis 150 und insbesondere technische C_{8/18}-Kokosfettsäuren eingesetzt, wobei gegebenenfalls eine Auswahl von cis/trans Isomeren wie elaidinsäurereiche C_{16/18}-Fettsäureschnitte von Vorteil sein kann. Sie sind handelsübliche Produkte und werden von verschiedenen Firmen unter deren jeweiligen Handelsnamen angeboten.
Die im Sinne der Erfindung einzusetzenden neuen Betainester der allgemeinen Formel (I), insbesondere aber die daraus herstellbaren Homo- und Copolymeren können zum Beispiel zur Herstellung von Haarkonditioniermitteln, hierbei insbesondere zur Herstellung von Haarshampoos, Haarkuren und Haarspülmitteln und dergleichen, verwendet werden. Sie beeinflussen hierbei in positiver Art und Weise sensorische Testgrößen wie zum Beispiel die Naßkämmbarkeit sowie je nach Art des eingesetzten Polymerisates auf Basis der Betainester der allgemeinen Formel (I) und geeigneter Comonomeren der allgemeinen Formel (II) Testparameter wie zum Beispiel Glanz oder Volumen.

Auch außerhalb der Kosmetik können die Betainester Anwendung finden insbesondere aufgrund ihrer Spaltbarkeit bei pH-Wertänderungen, so dass sich Schalter-Tenside mit der Möglichkeit gezielter Aktivierung bzw. Desaktivierung ergeben.

Für die Reinigung von Kraftfahrzeugen in Autowaschstraßen sind Betainester der allgemeinen Formel (I) ebenfalls von besonderem Interesse, da im Anschluss an die Kraftfahrzeugwäsche die Waschlauge mit Ölresten stark verschmutzt ist und ein Ölabscheider benutzt werden muss. Hierbei ist die pH-Wert-gesteuerte Spaltbarkeit der Betainester ebenfalls vorteilhaft.

Aufgrund ihres kationischen Charakters können Betainester der allgemeinen Formel (I) in Fußbodenpflegemitteln für. PVC- und andere Kunststoffböden eingesetzt werden, wobei sie den Glanz erhöhen und gleichzeitig bei der nächsten Anwendung wieder entfernbar sind, um Schichtaufbau auf der Fußbodenfläche zu vermeiden.

### Herstellungsbeispiele

Die Eduktmengen für die Darstellung der Chloressigsäureester errechnen sich auf Basis der von den einzusetzenden Alkoholen bestimmten OH-Zahlen. Dies gilt insbesondere für die als Alkoholkomponente eingesetzten Polyether. Die Reinheit der Chloressigsäureester wird über NMR-Spektroskopie, Säurezahl sowie den prozentualen Chlorgehalt im Produkt bestimmt.

Die Berechnung der einzusetzenden Menge an Chloressigsäureester für die Darstellung der Betainester erfolgt auf Basis des Chlorgehaltes. Zur Darstellung der erfindungsgemäßen ethylenisch ungesättigten Betainester der allgemeinen Formel (I) wird vorzugsweise käufliches N-(3-N',N'-Dimethylamino-propyl)acrylamid eingesetzt (Bezugsquelle Aldrich, 97 %, CAS 3845-76-9).

### Darstellung der ethylenisch ungesättigten Carbonsäureamidaminbetain-ester

### I. Chloressigsäure(cis-3-hexenyl)ester

In einem 250-ml-Vierhalskolben mit Rührer, Thermometer und Destillationsbrücke wurden unter Stickstoffatmosphäre 10 g (0,1 Mol) cis-3-Hexenol zusammen mit 10,4 g (0,11 Mol) Chloressigsäure für 2 h auf 120 °C erhitzt. Danach wurde die Temperatur auf 130 °C erhöht und für zwei weitere Stunden gerührt, wobei das entstehende Reaktionswasser kontinuierlich durch Destillation aus dem Reaktionsgemisch entfernt wurde. Nach insgesamt 4 h Reaktionszeit wurde ein Vakuum von 0,1 mbar angelegt, um überschüssige Chloressigsäure zu entfernen. Es wurden 17,6 g (0,1 Mol) einer gelblichen Flüssigkeit erhalten.
C₈H₁₃ClO₂ M = 176,7 g/Mol

Säurezahl: 9 mg (KOH)/g (Produkt), dies entspricht 0,22 % Cl aus der freien Chloressigsäure. Bei einem Gesamtchlorgehalt von 20,1 % entspricht dies einem Umsatz von 99 %.

¹H-NMR (400 MHz, CDCl₃): δ = 0,96 (t, ³J_{HH} = 7,53 Hz, 3H, CH₃), 2,03 (pseudo quintett, J_{HH} = 7,36 Hz, 2H, H-5'), 2,39 (pseudo quartett, J_{HH} = 7,05 Hz, 2H, H-2'), 4,03 (s, 2H, H-2), 4,15 (t, ³J_{HH} = 6,93 Hz, 2H, H-1') 5,27 (m, 1H, vinylisches H), 5,49 (m, 1H, vinylisches H) ppm.

¹³C-NMR (100,6 MHz, CDCl₃) δ = 13,52 (1C, CH₃), 19,97 (1C, CH₂), 25,92 (1C, CH₂), 40,32 (1C, CH₂Cl), 65,03 (1C, CH₂O), 122,54 (1C, C=C), 134,27 (1C, C=C), 166,89 (1C, C=O) ppm.

### II. N-(3-Acrylamidopropyl)-N,N-dimethyl-N-(cis-3-hexenyloxycarbonyl)-methylammoniumchlorid

Es wurden 17,6 g (0,1 Mol) Chloressigsäure(cis-3-hexenyl)-ester in einen 250-ml-Vierhalskolben mit Rührer, Thermometer und Rückflusskühler eingewogen. Nach Zugabe von 0,02 g (0,1 %) p-Methoxyphenol wurde unter Rühren auf die Reaktionstemperatur von 60 °C erhitzt und anschließend 15,6 g (0,1 Mol) N-(3-N',N'-Dimethylaminopropyl)acrylamid langsam zugetropft. Hierbei ist eine leichte Exothermie zu beobachten, wobei im weiteren Verlauf der Reaktion, die Temperatur auf 60 °C über die Zugabe des Acrylamidamins gehalten wurde. Nach vollendeter Zugabe des Acrylamidamins wurde weitere 4 h bei 60 °C gerührt, wobei eine deutliche Viskositätszunahme beobachtet wurde. Das Produkt liegt in Form einer braunen hochviskosen Masse vor.
C₁₆H₂₉ClN₂O₃ M = 332,92 g/Mol

Gesamtchlorgehalt: 10,6 %, davon Chlorid: 10,1 %, dies entspricht einem Umsatz von 93 %.

¹H-NMR (400 MHz, D₂O) : δ = 0,79 (t, ³J_{HH} = 7,51 Hz, 3H, CH₃), 1,84-1,96 (m, 4H), 2,31 (pseudo quartett, ³J_{HH} = 6,29 Hz, 2H, CH₂), 3,20-3,26 (m, 2H, NHCH₂), 3,31 (s, 6H, N(CH₃)₂), 3,43-3,49 (m, 2H, NCH₂), 4,13 (t, ³J_{HH} = 6,5 Hz, 2H, CH₂O), 4,15 (s, 2H, NCH₂C(O)), 5,19-5,27 (m, 1H, vinylisches H), 5,43-5,51 (m, 1H, vinylisches H), 5,64 (dd, ²J_{HH} = 9,52 Hz, ³J_{HH} = 2,05 Hz, 1H, vinylisches H), 6,05-6,16 (m, 1H, vinylisches H) ppm.

¹³C-NMR (100,6 MHz, D₂O) δ = 14,09, 20,63, 22,87, 26,29, 36,33, 52,58, 55,73, 61,67, 63,35, 67,0, 124,22, 128,1, 130,24, 136,03, 165,41, 169,04 ppm.

### III. Chloressigsäure(3,7-dimethyloct-6-en-1-yl)ester, Chloressigsäure(citronellol)ester

In einem 250-ml-Vierhalskolben mit Rührer, Thermometer und Destillationsbrücke wurden unter Stickstoffatmosphäre 15,6 g (0,1 Mol) Citronellol zusammen mit 10,4 g (0,11 Mol) Chloressigsäure für 3 h auf 120 °C erhitzt. Danach wurde die Temperatur auf 130 °C erhöht und für zwei weitere Stunden gerührt, wobei das entstehende Reaktionswasser kontinuierlich durch Destillation aus dem Reaktionsgemisch entfernt wurde. Nach insgesamt 4 h Reaktionszeit wurde ein Vakuum von 0,1 mbar angelegt, um überschüssige Chloressigsäure zu entfernen. Es wurden 22,3 g (0,096 Mol) einer gelblichen Flüssigkeit erhalten.
C₁₂H₂₁ClO₂ M = 232,7 g/Mol

Säurezahl: 8,4 mg (KOH)/g (Produkt), dies entspricht 0,0061 % Cl aus der freien Chloressigsäure.

Bei einem Gesamtchlorgehalt von 15,2 % entspricht dies einem Umsatz von 96 %.

¹H-NMR (400 MHz, CDCl₃): δ = 0,90 (d, ³J_{HH} = 6,56 Hz, 3H, CH₃), 1,11-1,22 (m, 1H), 1,27-1,37 (m, 1H), 1,41-1,50 22 (m, 1H), 1,53 (pseudo quartett, J_{HH} = 6,51 Hz, 1H), 1,58 (s, 3H, CH₃), 1,66 (s, 3H, CH₃), 1,65-1,73 (m, 1H), 1,87-2,03 (m, 2H), 4,02 (s, 2H, H-2), 4,15-4,26 (m, m, 2H, H-1') 5,06 (m, 1H, vinylisches H) ppm.

¹³C-NMR (100,6 MHz, CDCl₃) δ = 16,91 (1C, CH₃), 18,69 (1C, CH₂), 24,73 (1C, CH₃), 24,99 (1C, CH₃), 28,72 (1C), 34,66 (1C), 36,29 (1C), 40,26 (1C, CH₂Cl), 63,98 (1C, CH₂O), 123,89 (1C, C=C), 130,48 (1C, C=C), 161,71 (1C, C=O) ppm.

### IV. N-(3-Acrylamidopropyl)-N,N-dimethyl-N-(3,7-dimethyloct-6-en-1-oxy-carbonyl)methylammoniumchlorid

Es wurden 22 g (0,094 Mol) Chloressigsäure(citronellol)ester in einen 250-ml-Vierhalskolben mit Rührer, Thermometer und Rückflusskühler eingewogen. Nach Zugabe von 0,02 g (0,1 %) p-Methoxyphenol wurde unter Rühren auf die Reaktionstemperatur von 60 °C erhitzt und anschließend 14,7 g (0,094 Mol) N-(3-N',N'-Dimethylaminopropyl)acrylamid langsam zugetropft. Hierbei ist eine leichte Exothermie zu beobachten, wobei im weiteren Verlauf der Reaktion, die Temperatur auf 60 °C über die Zugabe des Acrylamidamins gehalten wurde. Nach vollendeter Zugabe des Acrylamidamins wurde weitere 4 h bei 60 °C gerührt, wobei eine deutliche Viskositätszunahme beobachtet wurde. Das Produkt liegt in Form einer hochviskosen Masse vor.
C₂₀H₃₇ClN₂O₃ M = 388,98 g/Mol

Gesamtchlorgehalt: 9,1 %, davon Chlorid: 8,7 %, dies entspricht einem Umsatz von 96 %.

¹H-NMR (400 MHz, CDCl₃) : δ = 0,87 (d, ³J_{HH} = 6,75 Hz, 3H, CH₃), 1,09-1,20 (m, 1H), 1,24-1,34 (m, 1H), 1,38-1,51 (m, 2H), 1,56 (s, 3H, CH₃), 1,64 (s, 3H, CH₃), 1,60-1,68 (m, 1H), 1,84-2,0 (m, 2H), 3,36-3,44 (m, 2H), 3,47 (s, 6H, N(CH₃)₂), 4,05-4,12 (m, 2H), 4,15-4,23 (m, 2H, CH₂O), 4,6 (s, 2H, NCH₂C(O)), 5,0-5,06 (m, 1H, vinylisches H_{Citronellol}), 5,55 (dd, ³J_{HH} = 10,11 Hz, ³J_{HH} = 2,04 Hz, 1H, vinylisches H-2), 6,24 (dd, ²J_{HH} = 17,05 Hz, ³J_{HH} = 2,04 Hz, 1H, vinylisches H-3a), 6,42 (dd, ²J_{HH} = 17,05 Hz, ³J_{HH} = 10,67 Hz, 1H, vinylisches H-3b), 8,47 (t, ³J_{HH} = 5,57 Hz, 1H, NH) ppm.

¹³C-NMR (100,6 MHz, CDCl₃) δ = 16,4 (1C, CH₃), 18,02, 21,52 (⁺NCH₂CH₂CH₂NC(O)), 23,96 (1C, CH₃), 24,45 (1C, CH₃), 28,05, 33,72, 34,86 (1C, CH₂NHC(O)), 35,53, 50,1 (2C, N(CH₃)₂), 59,96, 62,33, 64,0 (CH₂O), 122,97, 124,36 (C-2), 130,0, 130,14 (C-3), 163,30, 164,96 ppm.

### V. N-(3-Acrylamidopropyl)-N,N-dimethyl-N-(lauryl-PEG9-oxycarbonyl-methyl)ammoniumchlorid

Es wurden 65,74 g (0,1 Mol) Chloressigsäure(lauryl-PEG9)ester in einen 250-ml-Vierhalskolben mit Rührer, Thermometer und Rückflusskühler eingewogen. Nach Zugabe von 0,07 g (0,1 %) p-Methoxyphenol wurde unter Rühren auf die Reaktionstemperatur von 60 °C erhitzt und anschließend 15,6 g (0,1 Mol) N-(3-N',N'-Dimethylaminopropyl)acrylamid langsam zugetropft. Hierbei ist eine leichte Exothermie zu beobachten, wobei im weiteren Verlauf der Reaktion, die Temperatur auf 60 °C über die Zugabe des Acrylamidamins gehalten wurde. Nach vollendeter Zugabe des Acrylamidamins wurde weitere 4 h bei 60 °C gerührt, wobei eine deutliche Viskositätszunahme beobachtet wurde. Das Produkt ist eine hochviskose gelbe Masse.
C₄₀H₇₉ClN₂O₁₁ M = 799,52 g/Mol

Gesamtchlorgehalt: 4,4 %, davon Chlorid: 4,3 %, dies entspricht einem Umsatz von 98 %.

¹H-NMR (400 MHz, CDCl₃): δ = 0,84 (t, ³J_{HH} = 6,79 Hz, 3H, CH₃), 1,17-1,30 (m, 18H), 1,49-1,57 (m, 2H, CH₂), 1,98-2,16 (m, 2H, CH₂), 3,36-3,47 (m, 10H, CH₂, NHCH₂, N(CH₃)₂), 3,50-3,55 (m, 2H, CH₂), 3,56-3,64 (m, 30H), 3,65-3,70 (m, 2H, CH₂), 3,99-4,09 (m, 2H, NCH₂), 4,26-4,38 (m, 2H, CH₂O), 4,59-4,66 (m, 2H, NCH₂C(O)), 5,52-5,58 (m, 1H, vinylisches H), 6,2-6,28 (m, 1H, vinylisches H), 6,42 (dd, ²J_{HH} = 17,05 Hz, ³J_{HH} = 10,67 Hz, 1H, vinylisches H), 8,48-8,56 (m, 1H, NH) ppm.

¹³C-NMR (100,6 MHz, CDCl₃) δ = 13,08 (1C, CH₃), 21,56 (1C), 24,98 (1C), 28,23, 28,37, 28,50, 28,51, 28,54 (7C), 31,79 (1C), 35,05 (1C), 50,56 (2C), 60,26 (1C), 62,67 (1C), 64,11 (1C), 67,25, 68,95, 69,41, 70,30 (17C), 124,53 (1C), 130,46 (1C), 163,51 (1C), 165,15 (1C) ppm.

### VI. Chloressigsäure(butyl-PPG22**-PEG4*)ester

In einer 250-ml-Destillationsapparatur wurden 143,9 g (entsprechen 0,1 Mol basierend auf der OH-Zahl) Butyl-PPG22-PEG4-alkohol und 10,4 g (0,11 Mol) Chloressigsäure in Gegenwart von 0,39 g (2 mMol) p-Toluensulfonsäurehydrat unter Stickstoffatmosphäre für 4 h auf 130 °C erhitzt. Anschließend wurden im Vakuum (1 mbar) flüchtige Verunreinigungen sowie überschüssige Chloressigsäure entfernt. Es wurden 150,9 g einer gelb-orangen Flüssigkeit erhalten.
*PEG4 = -(CH₂-CH₂-O)₄- Polyethylenglycol
**PPG22 = -(CH₂-CH(CH₃)-O)₂₂- Polypropylenglycol
C₈₀H₁₅₉ClO₂₈ M = 1603,7 g/Mol

Säurezahl: 0,26 mg (KOH)/g (Produkt), dies entspricht 0,042 % Cl aus der freien Chloressigsäure. Bei einem Gesamtchlorgehalt von 2,21 % entspricht dies einem Umsatz von 98 %.

¹H-NMR (400 MHz, CDCl₃): δ = 0,87 (t, ³J_{HH} = 7,19 Hz, 3H, CH₃), 1,04-1,14 (m, 66H, PPG-CH₃), 1,27-1,37 (m, 2H, CH₂), 1,45-1,54 (m, 2H, CH₂), 1,98-2,10 (m, 2H, CH₂), 2,01 (s, 3H, CH₃), 3,25-3,72 (m, 82H), 4,02-4,05 (m, 1H, CH), 4,07 (m, 2H, CH₂), 4,29-4,34 (m, 1H, CH) ppm.

### VII. N-(3-Acrylamidopropyl)-N,N-dimethyl-N-(butyl-PPG22-PEG4-oxycarbonylmethyl)ammoniumchlorid

Es wurden 160,4 g (0,1 Mol) Chloressigsäure(butyl-PPG22-PEG4)ester in einen 500-ml-Vierhalskolben mit Rührer, Thermometer und Rückflusskühler eingewogen. Nach Zugabe von 0,2 g (0,1 %) p-Methoxyphenol wurde unter Rühren auf die Reaktionstemperatur von 60 °C erhitzt und anschließend 15,6 g (0,1 Mol) N-(3-N',N'-Dimethylaminopropyl)acrylamid langsam zugetropft. Hierbei ist eine leichte Exothermie zu beobachten, wobei im weiteren Verlauf der Reaktion, die Temperatur auf 60 °C über die Zugabe des Acrylamidamins gehalten wurde. Nach vollendeter Zugabe des Acrylamidamins wurde weitere 4 h bei 60 °C gerührt, wobei eine deutliche Viskositätszunahme beobachtet wurde. Es wurden 176 g einer orangen hochviskosen Flüssigkeit erhalten.
C₈₈H₁₇₅ClN₂O₂₉ M = 1759,85 g/Mol

Gesamtchlorgehalt: 2,2 %, davon Chlorid: 2,13 %, dies entspricht einem Umsatz von 97 %.

¹H-NMR (400 MHz, CDCl₃) : δ = 0,88 (t, ³J_{HH} = 7,51 Hz, 3H, CH₃), 1,05-1,15 (m, 66H, PPG-CH₃), 1,28-1,38 (m, 2H, CH₂), 1,47-1,56 (m, 2H, CH₂), 2,04-2,14 (m, 2H, CH₂), 3,24-3,70 (m, 92H), 4,0-4,14 (m, 2H), 4,28-4,36 (m, 2H), 4,58-4,64 (m, 1H, CH), 5,51-5,59 (m, 1H, vinylisches H), 6,18-6,28 (m, 1H, vinylisches H), 6,37-6,48 (m, 1H, vinylisches H), 8,10-8,28 (m, 1H, NH) ppm.

### Darstellung der polymeren Betainester

### VIII. Poly-N-(3-Acrylamidopropyl)-N,N-dimethyl-N-(cis-3-hexenyloxy-carbonyl)methylammoniumchlorid

### Homopolymerisation in Wasser:

Es wurden 16,7 g (0,05 Mol) des Acrylamidamin-Betainesters II in einen 250-ml-Vierhalskolben mit Rührer, Thermometer und Rückflusskühler unter Stickstoffatmosphäre eingewogen. Nach Zugabe von 50 ml destillierten Wassers wurde auf 90 °C erhitzt, wobei der Betainester sich langsam vollständig löste. Bei Erreichen der Reaktionstemperatur wurden 0,27 g (2 Mol-%) Kaliumperoxodisulfat, gelöst in 2 ml Wasser, unter Rühren über einen Zeitraum von 1 h zugetropft. Anschließend wurde für weitere 2 h bei 90 °C gerührt, und dann das Wasser im Vakuum abdestilliert. Es wurden 16,7 g eines hellgelben kristallinen Feststoffs erhalten.

### Homopolymerisation in Isopropanol:

Es wurden 14,42 g (0,041 Mol) des Acrylamidamin-Betainesters II in einen 250-ml-Vierhalskolben mit Rührer, Thermometer und Rückflusskühler unter Stickstoffatmosphäre eingewogen. Nach Zugabe von 13 g Isopropanol wurde auf 80 °C erhitzt, wobei der Betainester sich langsam vollständig löste und die Mischung einphasig wurde. Bei Erreichen der Reaktionstemperatur wurden 57 mg AMBN, gelöst in 0,92 g Isopropanol, unter Rühren über einen Zeitraum von 3 h zugetropft. Anschließend wurde für eine weitere Stunde bei 80 °C gerührt. Danach wurden zur Vervollständigung der Polymerisation 85 mg AMBN, gelöst in 0,5 g Isopropanol, über einen Zeitraum von 30 Minuten zugetropft und weitere 2 h bei 80 °C gerührt. Nach Abdestillieren des Lösungsmittels wurden 14,5 g des Homopolymerisates in Form eines hellgelben kristallinen Feststoffs erhalten.

¹H-NMR (400 MHz, D₂O): δ = 0,9-1,01 (m, 3H, CH₃), 1,25-1,90 (br, 3H, Polymerketten-CH/CH₂), 1,91-2,14 (br, 4H), 2,42-2,54 (br, 2H), 2,88-2,98 (br, 2H), 3,10-3,24 (br, 6H, N(CH₃)₂), 3,57-3,72 (br, 2H, NCH₂), 4,24-4,44 (br, 4H, CH₂O und NCH₂C(O)), 5,34-5,46 (br, 1H, vinylisches H), 5,58-5,70 (br, 1H, vinylisches H) ppm.

¹³C-NMR (100,6 MHz, D₂O) δ = 14,0 (br, 1C, CH₃), 20,4 (br, 1C), 22,8 (sehr breit, 1C), 25,2 (br, 1C), 26,1 (br, 1C), 36,0 (sehr breit, 1C), 42,0 (sehr breit, 1C), 50,8 (br, 2C, N(CH₃)₂), 61,1 (br, 1C, NCH₂), 63,7 (br, 1C, CH₂O), 65,6 (br, 1C, NCH₂C(O)), 122,2 (br, 1C, CH=CH), 135,1 (br, 1C, CH=CH), 164,8 (br, 1C, Ester C=O), 175,3 (br, 1C, Amid CO) ppm.

### IX. Poly- [N-(3-Acrylamidopropyl)-N,N-dimethyl-N-(cis-3-hexenyloxy-carbonyl)methylammoniumchlorid-co-acrylsäuremethylester]

### Copolymerisation in Isopropanol:

Zu 27 g Isopropanol in einem 250-ml-Vierhalskolben mit Rührer, Thermometer und Rückflusskühler wurden bei 80 °C unter Stickstoffatmosphäre gleichzeitig Lösungen des Acrylamidamin-Betainesters II (13,35 g, 0,04 mol in 27 g Isopropanol) und des Radikalstarters AMBN (40 mg in 5,2 g Acrylsäuremethylester) über einen Zeitraum von 80 min zugetropft. Anschließend wurden zur Vervollständigung der Polymerisation weitere 60 mg AMBN, gelöst in 5 g Isopropanol, über einen Zeitraum von 30 Minuten zugetropft und weitere 2 h bei 80 °C gerührt. Nach Abdestillieren des Lösungsmittels wurden 18,6 g des Copolymerisates in Form eines hellgelben kristallinen Feststoffs erhalten.

¹H-NMR (400 MHz, CDCl₃) : δ = 0,95 (t, ³J_{HH} = 7,54 Hz, 3H, CH₃), 1,22-1,96 (br, 3H, Polymerketten-CH/CH₂), 2,02 (pseudo quintett, J_{HH} = 7,44 Hz, 2H), 2,04-2,38 (br, 3H), 2,4 (m, br, 2H), 2,8-3,0 (br, 2H), 3,10-3,44 (br, 2H), 3,44-3,76 (br, 8H), 3,80-4,10 (br, 3H, OCH₃), 4,10-4,20 (br, 2H), 4,60-5,04 (br, 2H), 5,20-5,30 (br, 1H, vinylisches H), 5,45-5,55 (br, 1H, vinylisches H) 8,0-9,0 (br, 1H, NH) ppm.

¹³C-NMR (100,6 MHz, CDCl₃) δ = 13,84 (1C, CH₃), 20,25 (1C), 22,7 (sehr breit, 1C), 26,0 (br, 1C), 26,1 (br, 1C), 26,2 (br, 1C), 35,8 (sehr breit, 1C), 40,9 (sehr breit, 1C), 42,6 (sehr breit, 1C), 50,9 (br, 2C, N(CH₃)₂), 51,4 (br, 1C, OCH₃), 60,9 (br, 1C, NCH₂), 63,5 (br, 1C, CH₂O), 65,5 (br, 1C, NCH₂C(O)), 122,1 (br, 1C, CH=CH), 135,0 (br, 1C, CH=CH), 164,5 (br, 1C, Ester C=O), 166,3 (br, 1C, Ester C=O), 174,9 (br, 1C, Amid CO) ppm.

### X. Poly-N-(3-Acrylamidopropyl)-N,N-dimethyl-N-(3,7-dimethyloct-6-en-1-oxy-carbonyl)methylammoniumchlorid

Die Darstellung des Homopolymeren erfolgt analog der Synthese (in Isopropanol als Lösungsmittel) von VIII.

¹³C-NMR (100,6 MHz, CDCl₃) δ = 17,6 (1C, CH₃), 19,2 (br, 1C), 23,0 (sehr breit, 2C), 25,2 (br, 1C), 25,6 (br, 1C), 29,4 (br, 1C), 35,0 (br, 1C), 36,3 (br, 1C), 36,8 (sehr breit, 1C), 43,0 (sehr breit, 1C), 51,0 (br, 2C, N(CH₃)₂), 61,3 (br, 1C, NCH₂), 64,0 (br, 1C, CH₂O), 65,1 (br, 1C, NCH₂C(O)), 124,2 (br, 1C, CH=CH), 131,3 (br, 1C, CH=CH), 164,9 (br, 1C, Ester C=O), 175,7 (br, 1C, Amid CO) ppm.

### XI. Poly- [N- (3-Acrylamidopropyl) -N,N-dimethyl-N- (3, 7-dimethyloct-6-en-1-oxy-carbonyl)methylammoniumchloridco-acrylsäuremethylester]

Die Synthese des Copolymeren erfolgt analog der Herstellung von IX.

### Anwendungsbeispiele

Für die anwendungstechnische Beurteilung werden Haartressen, die für sensorische Tests verwendet werden, durch eine Dauerwellbehandlung und eine Bleichbehandlung standardisiert vorgeschädigt. Dazu werden friseurübliche Produkte verwendet.

Materialien
- Dauerwell-Flüssigkeit ("ondi", Wella)
- Fixierung ("neutrafix", Wella)
- Blondierpulver ("blondor special", Wella)
- H₂O₂ ("Welloxyd 9%", Wella)
- Shampoo ohne Pflegeanteil (Natriumlaurylethersulfat (12 % WAS), NaCl verdickt)
- Bechergläser
- Haar-Färbe-Pinsel

Die Behandlung wird in der folgenden Reihenfolge durchgeführt:

### I. Dauerwell-Behandlung

Die Haartressen werden mit der Dauerwellflüssigkeit angefeuchtet (Gewichtsverhältnis Haar : Flüssigkeit = 1 : 2). Nach einer Einwirkzeit von 15 Minuten bei Raumtemperatur in einem abgedeckten Becherglas wird die Dauerwellflüssigkeit 2 Minuten sorgfältig ausgespült. Anschließend werden die Haarsträhnchen mit einem Handtuch sanft ausgedrückt. Die Fixierung (Verhältnis Haar : Flüssigkeit = 1 : 2) hat eine Einwirkzeit von 10 Minuten bei Raumtemperatur. Anschließend wird die Fixierung sorgfältig 2 Minuten ausgespült. Die Haare werden anschließend über Nacht bei Raumtemperatur getrocknet.

### II. Bleich-Behandlung

Das Blondierpulver und das H₂O₂ werden zu einer Paste (Gewichtsverhältnis Pulver : H₂O₂ = 2 : 3) verarbeitet. Dann wird die Paste sofort auf die dauergewellten Haare mit einem Pinsel aufgetragen. Die Einwirkzeit beträgt 30 Minuten bei Zimmertemperatur. Anschließend wird die Blondierpaste unter fließendem Wasser 2 Minuten ausgespült. Dann wird das Haar mit einem Shampoo ohne Conditioner eine Minute gewaschen (Shampoomenge: 0,5 ml/ Hairtress) und dann für eine Minute ausgespült. Bevor die vorgeschädigten Haarsträhnchen für Sensorik-Tests verwendet werden, werden sie über Nacht bei Raumtemperatur getrocknet.

### Testrezeptur

Die konditionierenden Produkte werden in einer einfachen Haarspülung mit folgendem Aufbau getestet:

| **Produkt** | **Gewichtsanteile** |
|---|---|
| TEGINACID® C (INCI:Ceteareth-25) | 0,5 % |
| TEGO® Alkanol 16 (Cetyl Alcohol) | 2,0 % |
| "Conditioner" | 2,0 % |
| Wasser | ad. 100 % |
| Zitronensäure | ad. pH 4,0 ± 0,3 |

| | |
|---|---|
| ® Marke der Firma Goldschmidt AG | |

Als "Conditioner" sind die in den Herstellbeispielen und Vergleichsbeispielen erhaltenen Produkte bezeichnet.

Standardisierte Behandlung von vorgeschädigten Haarsträhnen mit konditionierenden Proben

Die wie oben beschrieben vorgeschädigten Haarsträhnchen werden wie folgt mit der oben beschriebenen konditionierenden Spülung behandelt:
Die Haarsträhnen werden unter fließendem, warmem Wasser benetzt. Das überschüssige Wasser wird leicht von Hand ausgedrückt, dann wird die Spülung aufgebracht und sanft im Haar eingearbeitet (1 ml /Haarsträhne (2 g)). Nach einer Verweilzeit von 1 Minuten wird für 1 Minuten gespült. Vor der sensorischen Beurteilung wird das Haar an der Luft bei 50 % Luftfeuchtigkeit und 25 °C für mindestens 12 h getrocknet.

### Beurteilungskriterien

Die sensorische Bewertungen erfolgt nach Noten, die auf einer Skala von 1 bis 5 vergeben werden, wobei 1 die schlechteste und 5 die beste Bewertung ist.

### Nasskämmbarkeit

| | | |
|---|---|---|
| 5 | Grobe Zahnung [des Kamms] | Keine Knoten, das Haar ist leicht entwirrbar. |
| | feine Zahnung | Sehr leichtes Durchkämmen, kein Widerstand spürbar. |
| 4 | grobe Zahnung | Einzelne Knoten. Das Haar ist leicht entwirrbar. |
| | feine Zahnung | Leichtes Durchkämmen, geringer Widerstand spürbar. |
| 3 | grobe Zahnung | Wenige Knoten, geringer Widerstand. |
| | feine Zahnung | Etwas Widerstand spürbar, der nach mehrmaligem Kämmen nachlässt. |
| 2 | grobe Zahnung | Einige Knoten, merklicher Widerstand. |
| | feine Zahnung | Merklicher Widerstand, der nach mehrmaligem Kämmen nicht nachlässt. |
| 1 | grobe Zahnung | Viele Knoten, starker Widerstand. |
| | feine Zahnung | Sehr starker Widerstand, manchmal ist das Haar nicht durchkämmbar. |

### Nassgriff

| | |
|---|---|
| 5 | sehr glatt, weich aber trotzdem schön fest, griffig, nicht schmierig/klebrig (keine Rückstände fühlbar) |
| 4 | glatt und weich und/oder nur geringe Rückstände spürbar |
| 3 | glatt, etwas hart und/oder etwas Rückstände spürbar |
| 2 | hart und/oder merkliche schmierige, wachsige Rückstände |
| 1 | sehr hart, rau, stumpf und/oder extrem schmierig, klebrig (deutlich spürbare schmierige, wachsige Rückstände spürbar) |

### Trockenkämmbarkeit

| | | |
|---|---|---|
| 5 | grobe Zahnung | keine Knoten, das Haar ist leicht entwirrbar |
| | feine Zahnung | sehr leichtes Durchkämmen, kein Widerstand spürbar, das Haar lädt sich nicht auf |
| 4 | grobe Zahnung | einzelne Knoten. Das Haar ist leicht entwirrbar |
| | feine Zahnung | leichtes Durchkämmen, geringer Widerstand spürbar, Haar lädt sich minimal auf |
| 3 | grobe Zahnung | wenige Knoten, geringer Widerstand |
| | feine Zahnung | etwas Widerstand spürbar, der nach mehrmaligem Kämmen nachlässt, Haar lädt sich wenig auf |
| 2 | grobe Zahnung | einige Knoten, merklicher Widerstand |
| | feine Zahnung | merklicher Widerstand, der nach mehrmaligem Kämmen nicht nachlässt, Haar lädt sich auf |
| 1 | grobe Zahnung | viele Knoten, starker Widerstand |
| | feine Zahnung | sehr starker Widerstand, manchmal ist das Haar nicht durchkämmbar, Haar lädt sich deutlich auf |

### Trockengriff

| | |
|---|---|
| 5 | sehr glatt, weich aber trotzdem fest, voll, griffig |
| 4 | glatt und weich |
| 3 | glatt, leicht hart und/oder leicht stumpf (Rückstände) |
| 2 | hart, etwas stumpf |
| 1 | rau, hart, trocken, stumpf (Rückstände) |

### Trocken-Aussehen

| | |
|---|---|
| 5 | extrem glänzend |
| 4 | glänzend |
| 3 | etwas glänzend |
| 2 | wenig glänzend, leicht stumpf |
| 1 | stumpf, kein Glanz |

### Volumen

Um das Volumen zu beurteilen, wird die Haarsträhne leicht geschüttelt, indem sie an der Klebestelle gehalten wird.

| | |
|---|---|
| 5 | lockerer, bauschiger Fall, ⌀ im Spitzenbereich rel. groß |
| 4 - 2 | Zwischenstufen |
| 1 | Haar hängt schwer herab, ⌀ unterhalb der Bündelung ähnlich dem Spitzenbereich |

Zur Ermittlung des elektrostatischen Verhaltens der Haarsträhne wird ein so genannter "Fly-Away-Test" durchgeführt. Dabei wird die Übertragung von elektrostatischer Ladung auf eine Haarsträhne durch Kämmen bestimmt. Gemessen wird die Aufspreizung der Haarsträhne durch elektrostatische Abstoßung der Haare untereinander. Zum Vergleich wird dieser Messwert für jedes Konditioniermittel relativ zum Messwert der Kontrollprobe angegeben (Reduktion der Aufspreizung gegenüber der Kontrollprobe).

In der folgenden Tabelle werden die Ergebnisse der sensorischen Beurteilung der wie oben beschrieben durchgeführten Behandlung der Haarstränchen mit erfindungsgemäßen Verbindungen bzw. Vergleichsbeispielen verglichen.

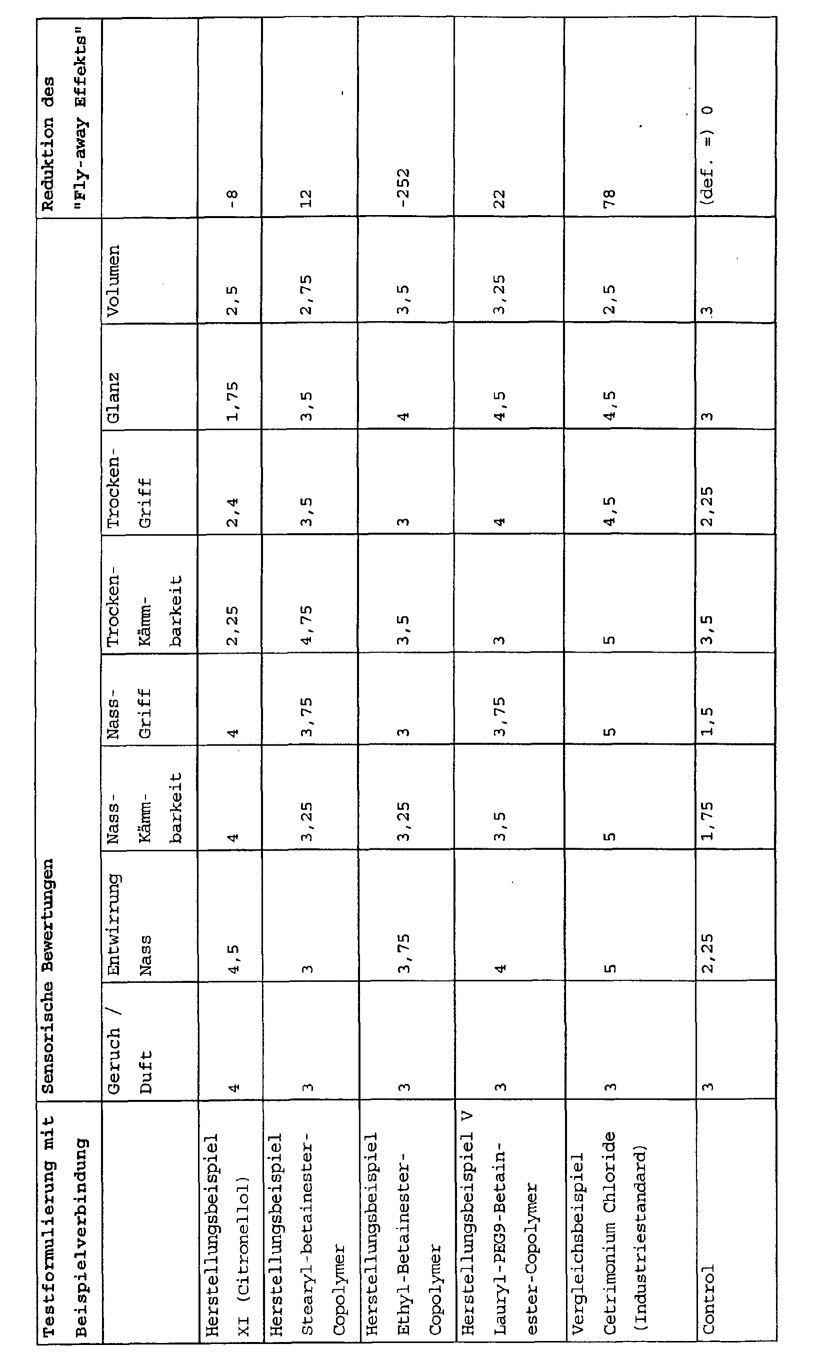

Es zeigt sich, dass die erfindungsgemäßen Verbindungen gute konditionierende Eigenschaften ergeben, die zum Teil denen des Industriestandards Cetrimonium Chlorid entsprechen und gegenüber den polyetherfreien Aminosiloxanen in der sensorischen Beurteilung erheblich bessere Bewertungen erhalten. Bei der Bewertung des Geruchs wird einzig das Herstellungsbeispiel XI besser als alle Vergleichbeispiele bewertet.

## Patentansprüche

1. Verbindungen der allgemeinen Formel (I) 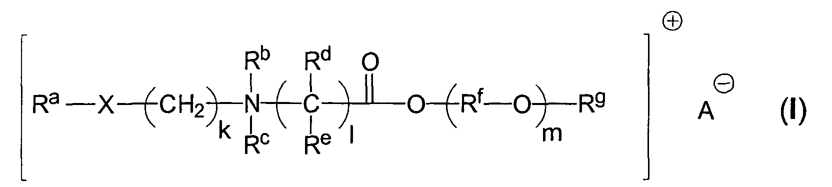 in denen
R^{a} ein ethylenisch ungesättigter, wenigstens eine Carbonylfunktion enthaltender Rest mit 1 bis 5 C-Atomen ist;
X ein Sauerstoffatom, -N(CH₃)- oder -NH- ist;
R^{b}, R^{c} unabhängig voneinander gegebenenfalls verzweigte, gegebenenfalls Heteroatomsubstituenten enthaltende Alkylreste mit 1 bis 4 C-Atomen sind;
die Reste
R^{d}, R^{e} unabhängig voneinander ausgewählt werden aus Wasserstoff (H), gegebenenfalls verzweigten Alkylresten mit 1 bis 4 C-Atomen, gegebenenfalls substituierten Aryl- oder Benzylresten sowie -CH₂COOH, -CH₂COOR, -CH₂CH₂COOH, -CH₂CH₂COOR;
R^{f} ein gegebenenfalls Mehrfachbindungen enthaltender verzweigter und/oder substituierter und/oder zyklischer Kohlenwasserstoffrest mit 1 bis 10 Kohlenstoffatomen ist oder ein Styrolrest ist oder ausschließlich aus Ethylen- oder Propylen- oder Butylen- oder Styrolresten aufgebaut ist oder ein die genannten Reste enthaltendes Block-Copolymer oder statistisch aufgebautes Copolymer ist;
R^{g} ein gegebenenfalls verzweigter, gegebenenfalls Doppelbindungen enthaltener, gegebenenfalls zyklischer Kohlenwasserstoffrest mit 1 bis 22 C-Atomen ist, wobei R^{g} ein gegebenenfalls verzweigter, gegebenenfalls Doppelbindungen enthaltener Kohlenwasserstoffrest ist, wenn m = 0 ist und R^{g} H sein kann, wenn m > 0 ist;
k, l unabhängig voneinander 1 bis 4 sind, wobei k vorzugsweise 2 oder 3 und l vorzugsweise 1 ist und
m einen Wert zwischen 0 bis 100, vorzugsweise 0 bis 40 hat;
A⁻ ein Anion ist.

2. Verbindungen gemäß Anspruch 1 in denen R^{a} -C(O)-CH=CH₂ ist.

3. Verbindungen gemäß den Ansprüchen 1 und 2 in denen R^{b} und R^{c} -CH₃ sind.

4. Verbindungen gemäß den Ansprüchen 1 bis 3 in denen R^{d} und R^{e} H sind.

5. Verbindungen gemäß den Ansprüchen 1 bis 4 in denen R^{f} ein Ethylenrest oder Propylenrest ist.

6. Verbindungen gemäß den Ansprüchen 1 bis 5 in denen R^{g} ein Alkylrest oder Alkenylrest mit 8 bis 18 C-Atomen ist.

7. Verbindungen gemäß den Ansprüchen 1 bis 6 in denen k 2 oder 3 und l 1 oder 2 ist.

8. Verbindungen gemäß den Ansprüchen 1 bis 7 in denen m = 0 und R^{g} ein Duftalkohol ist.

9. Verbindungen gemäß Anspruch 1 in denen R^{a} -C(O)-CH=CH₂ ist, R^{b} und R^{c} -CH₃ sind, R^{d} und R^{e} H sind, k = 3 ist, l = 1 ist, m = 0 ist und R^{g} ein Duftalkohol ist.

10. Verbindungen gemäß Anspruch 1 in denen R^{a} -C(O)-CH=CH₂ ist, R^{b} und R^{c} -CH₃ sind, R^{d} und R^{e} H sind, k = 3 ist, l = 1 ist, m = 0 bis 40 und R^{g} ein Alkylrest mit 1 bis 22 C-Atomen ist, R^{f} ein Styrolrest, ein Butylenrest, ein Propylenrest oder ein Ethylenrest ist oder ein ausschließlich aus Ethylen- oder Propylen- oder Butylenoder Styrolresten aufgebautes Homopolymer oder ein aus zwei oder mehreren dieser Reste aufgebautes Block-Copolymer oder statistisch aufgebautes Copolymer ist.

11. Verbindungen gemäß Anspruch 1 in denen R^{a} -C(O)-CH=CH₂ ist, R^{b} und R^{c} -CH₃ sind, R^{d} und R^{e} H sind, k = 3 ist, l = 1 ist, m = 0 bis 40 und R^{g} H ist, wobei R^{f} ein Styrolrest, ein Butylenrest, ein Propylenrest oder ein Ethylenrest ist oder ein ausschließlich aus Ethylen- oder Propylen- oder Butylen- oder Styrolresten aufgebautes Homopolymer oder ein aus zwei oder mehreren dieser Reste aufgebautes Block-Copolymer oder statistisch aufgebautes Copolymer ist.

12. Oligomere und polymere Verbindungen, hergestellt durch Copolymerisation von 0,5 bis 100 Mol-% eines polymerisierbaren Betainesters der allgemeinen Formel (I) und 0 bis 99,5 Mol-% eines ethylenisch ungesättigten Comonomeren der allgemeinen Formel (II) 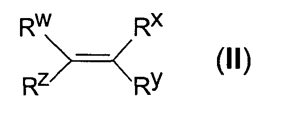 worin
R^{x} und R^{y} H sind, R^{w} H oder CH₃ ist und R^{z} ein wenigstens eine Carbonylgruppe enthaltender Rest -C(O)OR, -C(O)NR'R'' ist, wobei R, R' und R'' H oder gegebenenfalls Mehrfachbindungen enthaltende, lineare oder verzweigte und/oder zyklische und/oder substituierte und/oder Halogenatome enthaltende und/oder Heteroatome enthaltende und/oder Carbonylgruppen enthaltende Kohlenwasserstoffreste mit 1 bis 18 C-Atomen sind.

13. Oligomere und polymere Verbindungen, hergestellt durch Copolymerisation von 20 bis 70 Mol-% eines polymerisierbaren Betainesters der allgemeinen Formel (I), und 30 bis 80 Mol-% eines geeigneten ungesättigten Comonomeren der allgemeinen Formel (II).

14. Oligomere und polymere Verbindungen, hergestellt durch Copolymerisation von 40 bis 60 Mol-% eines polymerisierbaren Betainesters der allgemeinen Formel (I), und 60 bis 40 Mol-% eines ethylenisch ungesättigten Comonomeren der allgemeinen Formel (II).

15. Polymere gemäß den Ansprüchen 12 bis 14, enthaltend als Comonomere Verbindungen der Formel (II), worin R^{w} und R^{x} H sind, R^{y} und R^{z} eine Carbonylgruppe enthaltende Reste -C(O)OR, -C(O)NR'R'' sind, wobei R, R' und R'' H oder gegebenenfalls Mehrfachbindungen enthaltende, lineare oder verzweigte und/oder zyklische aliphatische oder aromatische und/oder substituierte und/oder Halogenatome und/oder Heteroatome enthaltende Kohlenwasserstoffreste mit 1 bis 18 C-Atomen sind.

16. Polymere gemäß den Ansprüchen 12 bis 14, enthaltend als Comonomere Verbindungen der Formel (II), worin R^{w}, R^{x} und R^{y} H sind und R^{z} ein gegebenenfalls halogenatom- und/oder heteroatomsubstituierter, lineare und/oder verzweigte Alkylsubstituenten enthaltender Aromat oder Heteroaromat ist.

17. Polymere gemäß den Ansprüchen 12 bis 14, enthaltend als Comonomere Verbindungen der Formel (II), worin R^{w}, R^{x} und R^{y} H sind und R^{z}-(CH₂)ₐ-OR''' ist, wobei R''' H oder ein gegebenenfalls Carbonylgruppen enthaltender Alkylrest mit 1 bis 22 C-Atomen oder ein ausschließlich aus Ethylenoder Propylen- oder Butylen- oder Styroloxid aufgebauter Polyether ist, der ein die genannten Reste enthaltendes, blockweise oder statistisch aufgebautes Copolymer darstellt und a 0 oder 1 ist.

18. Polymere gemäß den Ansprüchen 12 bis 14, enthaltend als Comonomere mindestens eine Verbindung aus der Gruppe
C₁-C₁₈-Acrylsäure-, Methacrylsäureester bzw. Maleinsäurehalb- oder -diester, wie Methylacrylat, Ethylacrylat, Propylacrylat, n-Butylacrylat, i-Propylacrylat, t-Butylacrylat, n-Hexylacrylat, Methylmethacrylat, Ethylmethacrylat, Propylmethacrylat, i-Propylmethacrylat, n-Butylmethacrylat, n-Hexylmethacrylat, n-Dodecylmethacrylat;
Polyethylenglykol- und Polypropylenglycolacrylate und -methacrylate;
C₂-C₁₈-Alkoxyester der Acryl- sowie Methacrylsäure wie Methoxyethylacrylat, Ethoxyethylacrylat, Methoxyethylmethacrylat, Ethoxyethylmethacrylat, Methoxybutylacrylat, Methoxybutylmethacrylat, Ethoxybutylacrylat, Ethoxybutylmethacrylat;
C₂-C₈-Hydroxyalkylacrylate und -methacrylate wie Hydroxyethylacrylat, Hydroxyethylmethacrylat, Hydroxypropylacrylat, Hydroxypropylmethacrylat, Hydroxybutylacrylat, Hydroxybutylmethacrylat;
Vinylverbindungen wie Styren, α-Methylstyren, Vinyltoluen, p-Chlorstyren, Vinylester wie Vinylacetat oder Vinylether, halogenierte Vinylverbindungen', Allylvinylketon, 4-Vinylpyridin, N-Vinylpyrrolidon, Vinylimidazol;
Amide der Acryl- und Methacrylsäure wie zum Beispiel Methacrylamid, N,N-Dimethylacrylamid, N,N-Dimethylmethacrylamid, N,N-Dimethylaminopropylacrylamid, N,N-Dimethylaminopropylmethacrylamid, Diallyldimethylammoniumchlorid (DADMAC);
Allylverbindungen.

19. Haarkonditioniermittel zur Herstellung von Haarshampoos. Haarkuren und Haarspülmitteln, enthaltend eine oder mehrere der polymerisierbaren und/oder polymeren Verbindungen der Ansprüche 1 bis 18.
